(19) Europäisches Patentamt
European Patent Office
Office européen des brevets

(11) **EP 0 947 831 A1**

(12) **EUROPEAN PATENT APPLICATION**
published in accordance with Art. 158(3) EPC

(43) Date of publication:
06.10.1999 Bulletin 1999/40

(51) Int. Cl.$^6$: **G01N 33/543**, G01N 33/53

(86) International application number:
PCT/JP97/04117

(21) Application number: 97912430.2

(22) Date of filing: 12.11.1997

(87) International publication number:
WO 98/22823 (28.05.1998 Gazette 1998/21)

(84) Designated Contracting States:
AT BE CH DE ES FR GB IT LI NL

(30) Priority: 15.11.1996 JP 30503396

(71) Applicant: DAINABOT CO., LTD.
Tokyo 106 (JP)

(72) Inventors:
• KOBAYASHI, Eiji,
Dainabot Co., Ltd.
Res & Dev cent
Matsudo-shi Chiba-ken 271 (JP)

• TAKEDA, Katsumichi,
Dainabot Co., Ltd.
Matsudo-shi Chiba-ken 271 (JP)

(74) Representative:
Modiano, Guido, Dr.-Ing. et al
Modiano, Josif, Pisanty & Staub,
Baaderstrasse 3
80469 München (DE)

(54) **WELLED PLATE FOR IMMUNOLOGICAL ANALYSIS**

(57) The object is to provide a well plate having wells to which a reagent has been added in advance and which permits storage and transportation.

The well plate for immunoassay includes wells, wherein each of the wells contains a dry reagent and is sealed with a desiccant-containing film at the upper surface of the well plate, is provided.

**Description**

Detailed Description of the Invention

Technical Field of the Invention

The present invention relates to well plates for immunoassay.

Prior Art

A variety of well plates such as 96-well plates have been used as reservoirs of samples in agglutination reactions making use of erythrocytes or colored beads or in immunochromatography making use of a strip. In those assay methods, reagents are added to each well of a well plate when samples are assayed. Accordingly, if a predetermined amount of a certain reagent were added to each well in advance, the assay procedure would be simplified. A well plate is also used as a solid phase in enzyme immunoassay (EIA), fluoroimmunoassay (FIA), chemiluminescence immunoassay (CLIA), etc. Weld plates onto which an antigen, antibody, etc. has been immobilized must be sealed within a bag that contains a dehumidifying agent so as to prevent the antigen, antibody, or other reagent from being deteriorated due to humidity. However, such bags impose considerable costs.

Object of the Invention

Accordingly, an object of the present invention is to provide a well plate which comprises wells wherein an reagent has been added to each of the wells in advance, and which permits storage and transportation.

Means to Attain the Object

By placing a dry reagent in each well of a well plate, and by subsequently sealing each well with a desiccant-containing film at the top surface of the well plate, the above-mentioned goal to provide a well plate that can be stored and transported is attained.

Modes for Carrying Out the Invention

Accordingly, the present invention is directed to a well plate for immunoassay which comprises wells, wherein each of the wells contains a dry reagent and is sealed with a desiccant-containing film at the upper surface of the well plate.

Any type of well plate that has conventionally been used may be used as the well plate of the present invention. In most cases, 96-well plates are used. A 96-well plate is formed of 12 rows of 8 wells. Well plates that can be used in sub-plates of blocks each containing 8 wells or 12 wells may also be used as the well plate of the present invention.

The shape of each well may be V-shaped, V-shaped, flat-bottomed, etc., in accordance with end uses. U-shaped wells and V-shaped wells are suitable for visual observation of the extent to which sensitized erythrocytes or colored beads agglutinate after immunological reaction. Flat-bottomed wells are suitable for the measurement of transmitted light, and are used in EIA or colloid agglutination. Thus, there have been known a variety of well plates for performing immunoassay, and therefore, the corresponding diameters and depths differ, the number of wells differs, and units by which wells are separated differ. In the present invention, however, no limitation is imposed on the shape or other features of well plates, and any type of well plate can be used.

The reagent that is added to each well in advance and dried is a reagent to be used in an immunoassay. If the immunological reaction is an agglutination reaction, the reagent that is added to wells in advance and dried comprises erythrocytes or colored beads that have been sensitized with an antigen or antibody. In passive hemagglutination (PHA) in which erythrocytes are used as carriers, antigen-sensitized erythrocytes are used, and agglutination of erythrocytes indicates the presence of a specific antibody against the sensitized antigen. In reverse hemagglutination (RPHA), antibody-sensitized erythrocytes are used, and agglutination of the sensitized erythrocytes is observed to thereby detect the presence of the antigen in the sample or to quantitatively determine the amount of the antigen. Agglutination assay in which certain colored beads are used instead of erythrocytes is also widely performed. As colored beads, there are used polystyrene beads, gelatin beads, liposomes, metal particles, and non-metal particles. In an agglutination reaction, colored beads to which an antigen or antibody has been bound, or erythrocytes sensitized with an antigen or antibody are dispensed into the wells and dried.

When the wells are reaction vessels or sample reservoirs for performing immunochromatography, the reagent which is added to the wells in advance and dried comprises antigens or antibodies that have been labeled with an enzyme or small colored particles. Examples of material for small colored particles include polystyrene, gelatin, liposomes, metals, non-metals, etc. Immunochromatography assay is performed by adding a sample liquid to wells to thereby form a complex comprising an antigen (or antibody) in the sample liquid and a labeled antibody (or antigen) that has been added to the wells and dried in advance, and by subsequently transferring the reaction mixture within a strip carrier by capillarity to cause a reaction between the complex and an immobilized antibody (or antigen) that has been immobilized on the detection position of the strip carrier in advance. Thus, the labeled substance that has been accumulated at the detection site is detected.

In EIA, FIA, CLIA, or the like, the reagent that is added to wells and dried in advance is an antigen or antibody that has been immobilized on walls of the wells in advance. Immobilization is generally performed through adsorption or covalent bonding. Moreover, by use of a labeled antigen or labeled antibody as a reagent to be added and dried in advance, the frequency of dispensing the reagent into the wells in EIA or other immunoassays can be reduced to thereby simplify the assay.

Drying is performed after a predetermined amount of a reagent solution has been added to each well of the well plate through freeze drying or drying in vacuo.

As described above, in EIA, FIA, CLIA, etc., an antigen or antibody is immobilized on the wall of a well. In most cases, this can be done through the following process. A predetermined amount of an antigen solution or antibody solution is dispensed into each well of a plastic plate made, for example, of polystyrene (PS), polyethylene (PE), or polypropylene (PP). After the well is allowed to stand for a predetermined period, the solution in the well is discarded, and the well is washed with a washing solution. Subsequently, the well plate is dewatered, and dried by the use of a desiccator or similar means. It is also possible to introduce a functional group to the material of the well plate to cause a reaction with the functional group of the reagent to thereby establish a linkage between the material and the reagent through covalent bonding. It is widely practiced that the covalent bonding is mediated by a linker. Subsequently, the immobilized antigen or antibody is dried.

The top surface of the wells of a well plate to which the dry reagent has been bound or added are sealed with a desiccant-containing film.

The desiccant-containing film is a multi-layered laminated film including (1) a desiccant-containing sheet which is formed by kneading a desiccant with a thermoplastic resin into a film, (2) an outer film which substantially does not permeate moisture, and (3) an inner film which is permeable to moisture and which is capable of adhering itself to the upper surface of the well plate when the desiccant-containing sheet is not able to be adhered to the upper surface of the well plate. An additional film for protecting the outer film may be superposed on the outer surface of the outer film.

The desiccant-containing film may be accompanied by a film containing an oxygen absorber. In cases where a film containing an oxygen absorber is included, the outer film substantially does not permeate moisture and oxygen, and the inner film is moisture- and oxygen-permeable.

The desiccant-containing sheet may be prepared through a conventional film-making method by kneading a dessicant such as $CaCl_2$, silica gel, molecular sieve, $SiO_2$, alumina, or zeolite into a thermoplastic polymer resin which permeates moisture such as PE, PP, or PS. Examples of commercially available desiccant-containing sheets include "Moisture Guard" (by Toyo Seikan K.K.) and "Highsheet Dry Film" (by Maruya-Kakoki K.K.).

The film containing an oxygen absorbent may be prepared through a conventional film-making method by kneading an oxygen absorbent such as active iron oxide or pyrogallol into a thermoplastic polymer resin which permeates oxygen such as PE, PP, or PS. An example of a commercially available sheet containing an oxygen absorbent is "Oxyguard" (by Toyo Seikan K.K.).

Examples of an outer film include PE film having a thickness of not less than 300 $\mu$m, PP film having a thickness of not less than 300 $\mu$m, polyvinylidene chloride (PVDC) film having a thickness of not less than 10 $\mu$m, and aluminum film having a thickness of not less than 7 $\mu$m. Also, examples of a film that substantially does not permeate moisture and oxygen include PVDC film having a thickness of not less than 15 $\mu$m, saponified products of polyvinyl alcohol having a thickness of not less than 15 $\mu$m, and aluminum film having a thickness of not less than 7 $\mu$m.

Examples of an inner film include PE film, PP film, or PS film, all having a thickness of not more than 30 $\mu$m, and film prepared from PE, PP, or PS as a major component and a suitable amount of auxiliary substances. The material of the inner film is determined in consideration of heat sealing properties with a well plate and ease of peeling after heat sealing.

Examples of the film for protecting the outer film include polyester (PET) film having a thickness of 12 $\mu$m, and nylon film having a thickness of 12 $\mu$m.

The wells are sealed with the desiccant-containing film so that the film tightly contacts the upper surface of the well plate. Although the film tightly contacts the well plate, the film must be able to be peeled off upon use.

The tight contact may be achieved by use of glue or heat sealing. As described above, the presence of an inner film is not necessary if the desiccant-containing sheet can be heat-sealed. In most cases where glue is used, glue is directly applied to a desiccant-containing sheet. In such cases, an inner film is not necessary.

In order for the film to be heat-sealed so as to be peeled off later, the inner surface of the desiccant-containing film and the upper surface of the well plate are made of different thermoplastic resins. Alternatively, the inner surface of a desiccant-containing film may be coated with a hot-melt agent.

When a desiccant-containing film is glued, the glue may be of the rubber type, acrylic type, or vinyl polymer ether type.

As the materials of the well plate of the present invention, in addition to PS, low moisture-permeable materials such as PE, PP, PET, polyvinyl chloride, etc. may be used for the prevention of moisture permeation. Also, in order to prevent moisture permeation, the lower surface of the well plate may be vapor-deposited with metal such as aluminum or coated with PVDC-containing paints, or the thickness of the well plate may be increased. Vapor-deposition of aluminum does not require any special method.

Moreover, in order to prevent permeation of moisture from the lower surface or side surfaces of a well plate, the lower surface or side surfaces of the well plate may be heat-sealed with an aluminum-foil-containing film comprising, for example, a lamination of (PE or PP film having a thickness of 70 $\mu$m)/(aluminum foil having a thickness of 7 $\mu$m)/(PET having a thickness of 12 $\mu$m) . Heat sealing is preferably performed so that the film is easily peeled off upon use.

To prevent the permeation of oxygen, it is advantageous to change the material or thickness of the well plate, or to modify the lower surface or side surfaces of the well plate.

In addition, in order to completely isolate the well plate from moisture and oxygen, a single well plate or a plurality of well plates may be put in a bag such as one made of aluminum foil.

When the well plate of the present invention is used in immunoassay, the desiccant-containing film is peeled off, a sample solution containing an analyte or a control solution is added to each well, and an ordinary immunoassay method is performed.

Examples

Example 1

To each well of a "Fastec U Plate" (FUJIREBIO INC.), a 25 $\mu$L aliquot of a solution prepared by dissolving a single vial of anti-HBsAg mouse monoclonal antibody/selenium colloid conjugate ("Dynascreen HBsAg" by Dainabot) in 1.5 mL of purified water was dispensed by use of a pipette, to thereby prepare a well plate having wells each containing a reagent. The well plate was subjected to preliminary freezing at -50°C for 30 minutes. Subsequently, the well plate was freeze-dried by elevating the temperature from -50°C to -5°C over 120 minutes, maintaining the temperature at -5°C for 10 hours, elevating the temperature from -5°C to 40°C over 120 minutes, maintaining the temperature at 40°C for 120 minutes, lowering the temperature from 40°C to 25°C over 30 minutes, and then maintaining the temperature at 25°C for 30 minutes (Plate A).

Another well plate to which a reagent had been dispensed was placed in a vacuum desiccator, and air was gradually removed by a vacuum pump. After air was completely removed, the well plate was left to stand at room temperature (23-28°C) for 15 hours, to thereby dry in vacuo. After the reagent was visually confirmed to have come to complete dryness, the well plate was removed from the vacuum desiccator (Plate B).

Plate A and plate B were independently heat-sealed with a desiccant-containing film constituted by a lamination of 12 $\mu$m PET, 15 $\mu$m Al, and 110 $\mu$m desiccant-containing PE/PS sheet. The heat-sealing was performed on a heat plate at 165°C for 0.7 seconds. The desiccant-containing PE/PS sheet had been kneaded with zeolite in an amount of 8 g per m$^2$.

The desiccant-containing film that sealed each of plates A and B was peeled off, and 50 $\mu$L of serum from healthy humans to which 1.6, 3.1, 6.3, 12.5, or 25 ng/mL of HBsAg had been added was dispensed (n=2). After dispensing, the wells were left for 2 minutes and then their contents were thoroughly stirred. To each well, an anti-HBsAg mouse monoclonal antibody-immobilized sheet ("Dynascreen HBsAg," Dainabot) mentioned above was placed. The results were determined after 15 and 40 minutes.

Controls were prepared by dispensing, in each well of a "Fastec U Plate" that did not contain a reagent, 25 $\mu$L of a solution prepared by dissolving a single vial of the above-mentioned anti-HBsAg mouse monoclonal antibody/selenium colloid conjugate in 1.5 mL of purified water, and then adding to the well 25 $\mu$L of serum from healthy humans to which 1.6, 3.1, 6.3, 12.5, or 25 ng/mL of HBsAg had been added. To each of the control wells, the aforementioned anti-HBsAg mouse monoclonal antibody-immobilized sheet was added, and the results were similarly determined after 15 and 40 minutes.

As shown in Table 1, plates A and B both retained anti-HBsAg antibody activity of the anti-HBsAg mouse monoclonal antibody/selenium colloid conjugate.

Table 1

| | Determined after 15 min. | | | | | Determined after 40 min. | | | | |
|---|---|---|---|---|---|---|---|---|---|---|
| | 25 | 13 | 6.3 | 3.1 | 1.6 | 25 | 13 | 6.3 | 3.1 | 1.6 |
| Control | +W | +/- | - | - | - | 2/+ | + | +/ | +/= | - |
| | +W | +/- | +/= | - | - | 2/+ | + | +/- | +/= | - |
| Plate A (before heat-sealing) | +W | +/= | | - | - | + | +W | +/= | - | |
| | +W | +/= | - | - | - | 2/+ | + | +/- | +/= | - |
| Plate A (after heat-sealing) | +W | +/- | - | - | | + | +W | +/- | +/= | - |
| | +W | +/= | - | - | - | 2/+ | +W | +/= | - | - |
| Plate B (before heat-sealing) | +W | +/- | - | - | | 2/+ | + | +/- | +/= | |
| | +W | +/= | - | - | - | 2/+ | + | +/= | - | - |
| Plate B (after heat-sealing) | +W | +/- | - | - | - | 2/+ | + | +/- | - | - |
| | +W | +/= | +/= | - | | 2/+ | + | +/- | +/= | - |

Signal intensity when determined:
2+ > 2/+ > + > +W > +/- > +/= (positive up to here) > - (negative)

Plates that had been heat-sealed with desiccant-containing film were stored at 2-8°C or 30°C, and the anti-HBsAg antibody activity was checked when 1 month, 3 months, and 6 months had elapsed.

To each well of the Plate A that had been stored was dispensed 50 μL of serum from healthy humans to which 1.6, 3.1, 6.3, 12.5, or 25 ng/mL of HBsAg had been added (n=2).

Controls were prepared by dispensing, in each well of a "Fastec U Plate" that did not contain a reagent, 25 μL of a solution prepared by dissolving a single vial of the mentioned anti-HBsAg mouse monoclonal antibody/selenium colloid conjugate in 1.5 mL of purified water, and adding to the well 25 μL of serum from healthy humans to which 1.6, 3.1, 6.3, 12.5, or 25 ng/mL of HBsAg had been added. After dispensing, the wells were left for 2 minutes and then their contents were thoroughly stirred. To each well, the aforementioned anti-HBsAg mouse monoclonal antibody-immobilized sheet was placed. The results were determined after 15 and 40 minutes.

As shown in Table 2, the plate A that had been stored retained anti-HBsAg antibody activity of the anti-HBsAg mouse monoclonal antibody/selenium colloid conjugate at the same level as before storage.

Table 2

| | | Determined after 15 min. | | | | | Determined after 40 min. | | | | |
|---|---|---|---|---|---|---|---|---|---|---|---|
| | | 25 | 13 | 6.3 | 3.1 | 1.6 | 25 | 13 | 6.3 | 3.1 | 1.6 |
| Stored for 1 month | Control | +W | +/- | +/= | - | - | 2/+ | + | +/- | +/= | - |
| | | +W | +/- | +/- | - | - | 2/+ | +W | +/ | +/= | - |
| | Plate A | +W | +/- | - | - | - | + | +W | +/- | +/= | - |
| | Stored at 2-8°C | +W | +/- | +/= | - | - | 2/+ | +W | +/- | +/= | |
| | Plate A | +W | +/- | - | - | - | 2/+ | + | +/- | +/= | |
| | Stored at 30°C | +W | +/- | - | - | - | 2/+ | +W | +/- | | - |

Table 2 (continued)

| | | Determined after 15 min. | | | | | Determined after 40 min. | | | | |
|---|---|---|---|---|---|---|---|---|---|---|---|
| | | 25 | 13 | 6.3 | 3.1 | 1.6 | 25 | 13 | 6.3 | 3.1 | 1.6 |
| Stored for 3 months | Control | +W | +/- | +/- | - | - | 2/+ | + | +/- | +/= | - |
| | | +W | +/- | - | - | - | 2/+ | + | +/- | - | - |
| | Plate A | +W | +/= | - | - | - | + | +W | +/= | - | - |
| | Stored at 2-8°C | +W | +/= | - | - | - | 2/+ | + | +/- | +/= | - |
| | Plate A | +W | +/- | - | - | - | + | +W | +/- | +/= | - |
| | Stored at 30°C | +W | +/= | - | - | | 2/+ | +W | +/= | - | - |
| Stored for 6 months | Control | 1W | +/- | - | - | - | 2/+ | +W | +/ | - | - |
| | | +W | +/- | +/= | - | - | 2/+ | + | +/- | +/= | - |
| | Plate A | +W | +/- | 1/= | - | - | 2/+ | + | +/- | +/= | - |
| | Stored at 2-8°C | +W | +/= | - | - | - | 2/+ | + | +/- | +/= | - |
| | Plate A | +W | +/- | - | | - | + | +W | +/- | +/= | - |
| | Stored at 30°C | +W | +/= | - | - | - | 2/+ | + | +/- | +/= | - |

Signal intensity when determined:
2/+ > 2/+ > + > +W > +/- > +/= (positive up to here) > - (negative)

Example 2

Treponema pallidum (TP) soluble antigen was diluted with 0.4% sodium carbonate buffer (pH 9.8) so as to achieve a concentration of 0.2 μg/mL. The diluted antigen was dispensed in the wells of an "Immunoplate Polysolb F-96" (96-well flat-bottomed plate; by Nunc) in an amount of 90 μL per well. The openings of the wells were closed with a lid, and the plate was allowed to stand for 16 hours. The liquid in each well was discarded, and 200 μL of a washing solution (0.9% NaCl solution containing 0.1% Tween 20) was added to each well to wash the well. After washing was completed, the washing solution was discarded. The washing procedure was performed a total of three times. After moisture was absorbed by use of blotting paper, the plate was dried at room temperature for 2 hours in vacuo in a vacuum desiccator. The thus-obtained TP antigen-immobilized plate was heat-sealed with a desiccant-containing film in a manner similar to that described in Example 1.

Through use of plates that had or had not undergone heat sealing with a desiccant-containing film, a test was performed to check whether the activity of the sample (TP antigen) was retained. The desiccant-containing film that had sealed the wells of a well plate was peeled off, and 100 μL of a negative control (serum from healthy humans) or TP antibody-positive serum diluted with serum from healthy humans was dispensed to each well. The plate was incubated at 37°C for 1 hour. A washing liquid (200 μL) was added to each well for washing the well. The washing procedure was performed three times. Subsequently, a peroxydase-labeled anti-human IgG goat antibody liquid (100 μL) was added and the mixture was incubated at 37°C for 1 hour. Each well was washed three times with 200 μL of a washing liquid each time. A color developing solution for a substrate (100 μL, a 0.05% citrate buffer containing 0.1% 2,2'-azinobis-(3-ethylbenzothioazoline-6-sulfonic acid) (ABTS, product of Wako Pure Chemicals) and 0.05% of hydrogen peroxide) was added and the plate was incubated at 37°C for 30 minutes. Development of color was stopped by the addition of 100 μL of a reaction stopper solution (a solution containing 1% sodium fluoride and 2% citric acid). Within 10 minutes after development of color was stopped, absorbance at 405 nm was measured.

Through use of a plate that had not undergone heat-sealing with a desiccant-containing film, a similar test was performed, which plate represented a control.

The results are shown in Table 3. Even after the well plates were heat-sealed with desiccant-containing film, no reduction in the activity of TP antigen was observed.

Table 3

| | | Negative control | Diluted anti-TP-antibody-positive serum | | | | |
|---|---|---|---|---|---|---|---|
| | | | x6400 | x3200 | x1600 | x800 | x400 |
| Plate C | | 0.085 | 0.290 | 0.459 | 0.690 | 0.969 | 1.804 |
| | | 0.076 | 0.252 | 0.429 | 0.624 | 1.083 | 1.780 |
| Average | | 0.081 | 0.271 | 0.444 | 0.657 | 1.026 | 1.792 |
| Plate D | | 0.078 | 0.253 | 0.386 | 0.616 | 1.004 | 1.783 |
| | | 0.085 | 0.263 | 0.392 | 0.655 | 1.087 | 1.883 |
| Average | | 0.082 | 0.258 | 0.389 | 0.636 | 1.046 | 1.833 |

A heat-sealed Plate C was stored at 2-8°C or 30°C. Plate D that had not undergone heat-sealing was tightly sealed within a bag of aluminum foil together with 5 g of silica gel, and stored at 2-8°C. Following storage for 1 month, 3 months, and 6 months, the activity of the immobilized TP antigen was investigated.

To each well of the respective plates, 100 μL of a negative control (serum from healthy humans) or TP antibody-positive serum diluted with serum from healthy humans was added. The openings of the wells were closed with a lid, and the plate was incubated at 37°C for 1 hour.

A washing liquid (200 μL) was placed in each well for washing the well. The washing procedure was performed three times. Subsequently, a peroxydase-labeled anti-human IgG goat antibody liquid (100 μL) was added and the mixture was incubated at 37°C for 1 hour under a lid.

Each well was washed three times with 200 μL of a washing liquid per washing. A color developing solution for a substrate (100 μL, a 0.5% citrate buffer containing 0.1% ABTS (product of Wako Pure Chemicals) and 0.05% of hydrogen peroxide) was added, the mixture being covered with a lid, and the plate was incubated at 37°C for 30 minutes.

Development of color was stopped by the addition of 100 μL of a reaction stopper solution (a solution containing 1% sodium fluoride and 2% citric acid). Within 10 minutes after development of color was stopped, absorbance at 405 nm was measured. The results are shown in Table 4. Neither the well plate C or D, both of which had undergone storage, showed a reduction in activity of immobilized TP antigen.

Table 4

| | | Negative control | Diluted anti-TP antibody-positive serum | | | | |
|---|---|---|---|---|---|---|---|
| | | | x6400 | x3200 | x1600 | x800 | x400 |
| Stored for 1 month | Plate C | 0.090 | 0.235 | 0.334 | 0.526 | 1.023 | 1.790 |
| | Stored at 2-8°C | 0.092 | 0.271 | 0.422 | 0.685 | 1.131 | 1.852 |
| | Average | 0.091 | 0.253 | 0.378 | 0.606 | 1.077 | 1.821 |
| | Plate C | 0.098 | 0.273 | 0.423 | 0.675 | 1.089 | 1.931 |
| | Stored at 30°C | 0.099 | 0.306 | 0.459 | 0.661 | 1.159 | 1.686 |
| | Average | 0.099 | 0.290 | 0.441 | 0.668 | 1.124 | 1.809 |
| | Plate D | 0.095 | 0.256 | 0.402 | 0.601 | 1.011 | 1.855 |
| | Stored at 2-8°C | 0.099 | 0.299 | 0.410 | 0.622 | 1.112 | 1.665 |
| | Average | 0.097 | 0.278 | 0.406 | 0.612 | 1.062 | 1.760 |

Table 4 (continued)

| | | Negative control | Diluted anti-TP antibody-positive serum | | | | |
|---|---|---|---|---|---|---|---|
| | | | x6400 | x3200 | x1600 | x800 | x400 |
| Stored for 3 months | Plate C | 0.132 | 0.254 | 0.382 | 0.579 | 1.039 | 1.726 |
| | Stored at 2-8°C | 0.124 | 0.257 | 0.442 | 0.615 | 1.065 | 1.787 |
| | Average | 0.128 | 0.256 | 0.412 | 0.597 | 1.052 | 1.757 |
| | Plate C | 0.138 | 0.271 | 0.424 | 0.629 | 1.097 | 1.821 |
| | Stored at 30°C | 0.143 | 0.276 | 0.444 | 0.611 | 1.118 | 1.819 |
| | Average | 0.141 | 0.274 | 0.434 | 0.620 | 1.108 | 1.820 |
| | Plate D | 0.135 | 0.266 | 0.401 | 0.601 | 1.001 | 1.779 |
| | Stored at 2-8°C | 0.122 | 0.250 | 0.435 | 0.622 | 1.121 | 1.801 |
| | Average | 0.129 | 0.258 | 0.418 | 0.612 | 1.061 | 1.790 |
| Stored for 6 months | Plate C | 0.092 | 0.252 | 0.423 | 0.675 | 1.022 | 1.850 |
| | Stored at 2-8°C | 0.085 | 0.273 | 0.386 | 0.616 | 1.005 | 1.781 |
| | Average | 0.089 | 0.263 | 0.405 | 0.646 | 1.014 | 1.816 |
| | Plate C | 0.077 | 0.271 | 0.429 | 0.690 | 1.099 | 1.921 |
| | Stored at 30°C | 0.098 | 0.252 | 0.422 | 0.553 | 1.113 | 1.699 |
| | Average | 0.088 | 0.262 | 0.426 | 0.622 | 1.106 | 1.810 |
| | Plate D | 0.088 | 0.250 | 0.399 | 0.611 | 0.999 | 1.859 |
| | Stored at 2-8°C | 0.085 | 0.287 | 0.435 | 0.599 | 1.152 | 1.911 |
| | Average | 0.087 | 0.269 | 0.417 | 0.605 | 1.076 | 1.885 |

Effect of the Invention

As described above, according to the present invention, there has been provided a well plate having wells to which a reagent had been added in advance and which permits storage and transportation. Through use of the well plate, immunoassay procedure can be simplified.

**Claims**

1.   A well plate for immunoassay comprising wells, wherein each of the wells contains a dry reagent and is sealed with a desiccant-containing film at the upper surface of the well plate.

## INTERNATIONAL SEARCH REPORT

| International application No. |
| --- |
| PCT/JP97/04117 |

### A. CLASSIFICATION OF SUBJECT MATTER

Int. Cl$^6$  G01N33/543

According to International Patent Classification (IPC) or to both national classification and IPC

### B. FIELDS SEARCHED

Minimum documentation searched (classification system followed by classification symbols)

Int. Cl$^6$  G01N33/543, B65D85/00

Documentation searched other than minimum documentation to the extent that such documents are included in the fields searched

| | |
| --- | --- |
| Jitsuyo Shinan Koho | 1926 – 1997 |
| Kokai Jitsuyo Shinan Koho | 1971 – 1995 |
| Toroku Jitsuyo Shinan Koho | 1994 – 1997 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| Y | JP, 62-46262, A (Eiken Chemical Co., Ltd.), February 28, 1987 (28. 02. 87), Fig. 2 (Family: none) | 1 |
| Y | JP, 8-268481, A (Toppan Printing Co., Ltd.), October 15, 1996 (15. 10. 96), Claims; Par. No. (0013) (Family: none) | 1 |

☐ Further documents are listed in the continuation of Box C.   ☐ See patent family annex.

| | |
| --- | --- |
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| December 5, 1997 (05. 12. 97) | December 16, 1997 (16. 12. 97) |

| Name and mailing address of the ISA/ | Authorized officer |
| --- | --- |
| Japanese Patent Office | |
| Facsimile No. | Telephone No. |

Form PCT/ISA/210 (second sheet) (July 1992)